# EUROPÄISCHE PATENTSCHRIFT

(11) **EP 1 828 149 B1**
(45) Veröffentlichungstag und Bekanntmachung des Hinweises auf die Patenterteilung: **21.01.2015**
(21) Anmeldenummer: 05817378.2
(22) Anmeldetag: 01.12.2005
(51) Int. Cl.: C07D 263/32, A61K 31/41, A61P 3/00

(54) **VERFAHREN ZUR HERSTELLUNG VON OXAZOLEN DURCH KONDENSATION VON AROMATISCHEN ALDEHYDEN MIT ALFA-KETOXIMEN ZU N-OXIDEN UND NACHFOLGENDE REAKTION MIT AKTIVIERTEN SÄUREDERIVATEN**
METHOD FOR THE PRODUCTION OF OXAZOLES BY CONDENSING AROMATIC ALDEHYDES WITH A-KETOXIMES TO N-OXIDES AND THEN REACTING THE SAME WITH ACTIVATED ACID DERIVATIVES
PROCÉDÉ DE FABRICATION D'OXAZOLES PAR CONDENSATION D'ALDÉHYDES AROMATIQUES AVEC DES (A)-CETOXIMES AFIN DE PRODUIRE DES N-OXYDES ET RÉACTION CONSÉCUTIVE AVEC DES DÉRIVÉS D'ACIDE ACTIVÉS

(30) Priorität: 15.12.2004 DE 102004060227
(43) Veröffentlichungstag der Anmeldung: 05.09.2007
(73) Patentinhaber: Sanofi-Aventis Deutschland GmbH, 65929 Frankfurt am Main (DE)
(72) Erfinder: HOLLA, Wolfgang, 65779 Kelkheim (DE); HOERLEIN, Rolf-Ludwig, 60529 Frankfurt (DE); KULITZSCHER, Berndt, 97839 Steinmark (DE); LAUX, Wolfgang, 60599 Frankfurt (DE); STUEDEMANN, Thomas, 65779 Kelkheim (DE); TAPPERTZHOFEN, Christoph, 60385 Frankfurt (DE); SCHEFFER, Robert, J., H., 65926 Frankfurt am Main (DE)
(86) Internationale Anmeldenummer: PCT/EP2005/012800
(87) Internationale Veröffentlichungsnummer: WO 2006/066694

(56) Entgegenhaltungen:
- WO-A-02/16332
- WO-A-02/100403
- WO-A-2004/075815
- WO-A-2004/076426

## Beschreibung

Verfahren zur Herstellung von Oxazolen durch Kondensation von aromatischen Aldehyden mit α-Ketoximen zu N-Oxiden und nachfolgende Reaktion mit aktivierten Säurederivaten.

Die Erfindung betrifft ein Verfahren zur Herstellung von Oxazolen durch Kondensation von Aldehyden mit α-Ketoximen zu N-Oxiden in Form ihrer Salze oder als freie Basen und nachfolgende Reaktion mit aktivierten Säurederivaten zu Oxazolen in Form ihrer Salze oder als freie Basen, insbesondere die Kondensation zwischen aromatischen Aldehyden und α-Ketoximen gefolgt von der Umsetzung mit anorganischen Thionylhalogeniden oder organischen Sulfonylhalogeniden zu Chlormethyloxazolen.

Die Erfindung erlaubt die Herstellung von Oxazolen in hoher Ausbeute und grosser Reinheit. Oxazole stellen wertvolle Zwischenstufen bei der Synthese von pharmazeutisch wirksamen Stoffen, wie z. B. PPAR Agonisten, dar. Entsprechende Beispiele von PPAR Agonisten sind u.a. in WO 03/020269, WO 2004/075815, WO 2004/076447, WO 2004/076428, WO 2004/076426, WO 2004/076427, DE 102004039533.0, DE 102004039532.2, DE 102004039509.8 beschrieben. Letztere sind Arzneistoffe, die sowohl den Lipid- als auch den Glucosestoffwechsel positiv beeinflussen können.

Die Kondensation aromatischer Aldehyde mit α-Ketoximen zu N-Oxiden und die nachfolgende Umsetzung mit aktivierten Säurederivaten zu Oxazolen ist an sich bekannt.

In der Literatur werden für die Umwandlung der N-Oxide in die Oxazole die Reagentien Phosphor(III)-chlorid (PCl₃) und Phosphoroxychlorid (POCl₃) und in einer Variante Essigsäureanhydrid ((CH₃COO)₂O) beschrieben (Y. Goto, M. Yamazaki, M. Hamana, Chem Pharm Bull. 19 (1971) 2050, und dort zitierte Literatur). Diese Reagentien sind nicht breit anwendbar und führen oft zu keinen oder zu stark verunreinigten Produkten, die nur aufwändig, z. B. durch chromatographische Verfahren, mit niedrigen Ausbeuten in ausreichender Reinheit zu erhalten sind.

Die beschriebenen Reaktionsbedingungen verlangen die Isolation der N-Oxide. Für N-Oxide mit exothemem Zersetzungspotential stellt dies ein erhebliches Sicherheitsrisiko dar und verbietet die Verfahrensausübung im industriellen Maßstab.

Überraschend wurde nun gefunden, dass die Transformation der N-Oxide in die Halogenmethyloxazole unerwartet glatt mit hoher Ausbeute und großer Reinheit mit anorganischen Thionylhalogeniden oder organischen Sulfonylhalogeniden abläuft. Obwohl aufgrund der Ausführungen in der Literatur nicht zu erwarten, fallen Halogenmethyloxazole teilweise direkt aus der Reaktionsmischung in Form der freien Base oder als Salze sauber aus.
Unerwarteterweise konnte für N-Oxide mit exothermem Zersetzungspotential sowohl die sichere Herstellung in verdünnter Lösung als auch die weitere direkte Umsetzung der Lösung zu den Halogenmethyloxazolen erreicht werden.

Die Erfindung betrifft somit ein Verfahren zur Herstellung der Verbindungen der Formel IV - mittels Überführung von aromatischen Aldehyden der Formel I mit α-Ketoximen der Formel II über N-Oxide der Formel III, in Halogenmethyloxazole der Formel IV -, 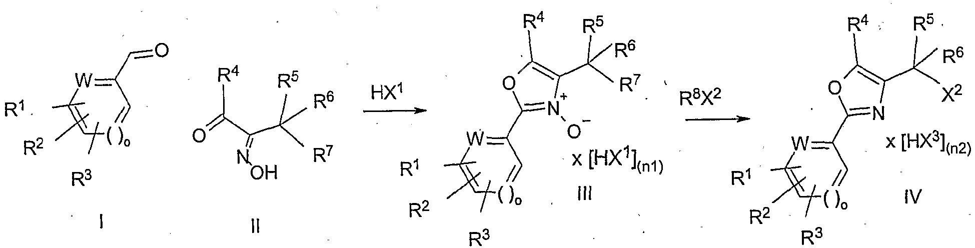 das dadurch gekennzeichnet ist, dass man die aromatischen Aldehyde der Formel I mit den α-Ketoximen der Formel II 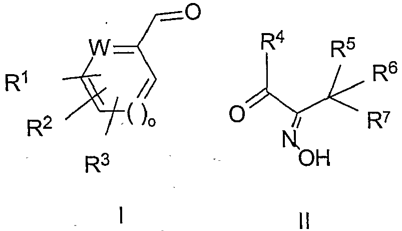 worin bedeuten:
- R¹: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl;
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann;
- R²: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind;
- R³: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind;
- W: CH, N, falls o = 1;
- W: O, S, NR12, falls o = 0;
- o: 0 oder 1;
- R12: H, (C1-C6)-Alkyl, (C1-C6)-Alkylen-Phenyl, Phenyl;
- R⁴: H, (C1-C8)-Alkyl, (C3-C8)-Cycloalkyl, (C1-C3)-Alkylen-(C3-C8)-Cycloalkyl, Phenyl, (C1-C3)-Alkylen-Phenyl, (C5-C6)-Heteroaryl, (C1-C3)-Alkylen-(C5-C6)-Heteroaryl oder (C1-C3)-Alkyl, das durch F ganz oder teilweise substituiert ist, oder COOR⁹, CONR(10)R(11);
wobei R⁹, R¹⁰ und R¹¹ wie oben definiert sind;
- R⁵ und R⁶: unabhängig voneinander
H, (C1-C8)-Alkyl, F, Cl, Br, I, O-(C0-C8)-Alkylen-H, O-(C6-C10)-Aryl, O-(C1-C4)-Alkylen-(C6-C10)-Aryl, COOR⁹, CONR¹⁰R¹¹, SH oder NR¹⁰R¹¹,
wobei R⁹, R¹⁰, R¹¹ wie oben definiert sind;
oder
- R⁵ und R⁶: gemeinsam

für (C4-C5)-Alkylen steht, wobei eine CH2-Gruppe durch O, S, NH, N-CH₃ oder N-Benzyl ersetzt sein kann;
- R⁷: H oder (C1-C8)-Alkyl;

in Gegenwart von Säuren HX¹, wie beispielsweise HCl, HBr, H₂SO₄, H₃PO₄, HOOCCF₃, HOOCCCl₃, HO₃SCF₃, HO₃SCH₃, HO₃SC₆H₅ , HO₃S-C₆H₄-p-CH₃, HOOCH,

in die N-Oxide der Formel III überführt, 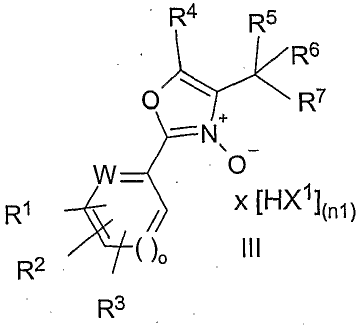 worin R¹, R², R³, R⁴, R⁵, R⁶, R⁷ und X¹ wie oben definiert sind und
- n1: 0, 1, ½ oder 1/3 bedeutet;
und diese anschließend
mit dem Reagens R⁸X² , welches bedeutet:
SOCl-Cl, SOBr-Br, CH₃SO₂-Cl, CF₃SO₂-Cl, C₆H₅SO₂-Cl, p-CH₃-C₆H₄-SO₂-Cl, CH₃SO₂-O₃SCH₃, CF₃SO₂-O₃SCF₃, C₆H₅SO₂-O₃SC₆H₅ oder p-CH₃-C₆H₄-SO₂-O₃S-C₆H₄-p-CH₃ ,
zu den Halogenmethyloxazolen der Formel IV umsetzt, 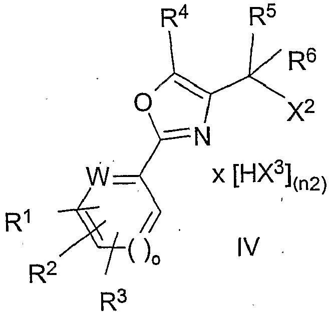 worin R¹, R², R³, R⁴, R⁵, R⁶ und X² wie oben definiert sind und
- X3: Cl, Br, CH₃SO₃, CF₃SO₃, C₆H₅SO₃, oder p-CH₃-C₆H₄-SO₃ und
- n2: 0 oder 1 bedeuten.

Bevorzugt betrifft die Erfindung ein Verfahren zur Herstellung der Verbindungen der Formel IV worin bedeuten:
W = CH und
o = 1.

Ferner betrifft die Erfindung bevorzugt ein Verfahren zu Herstellung der Verbindungen der Formel IV worin bedeuten:
- R1: H;
- R²: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert. ist durch F, Cl, Br, I, (C1-C4)-Alkyl, O-(C1-C4)-Alkyl oder CF₃;
wobei bedeuten:
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl;
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann;
- R³: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind.

Besonders bevorzugt betrifft die Erfindung ein Verfahren zu Herstellung der Verbindungen der Formel IV worin bedeuten:
- R1: H;
- R²: H;
- R³: H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann.

Besonders bevorzugt betrifft die Erfindung ferner ein Verfahren zu Herstellung der Verbindungen der Formel IV worin bedeuten:
- R¹, R², R³: unabhängig voneinander H, (C1-C6)-Alkyl, F, Cl, Br, I, O-(C0-C8)-Alkylen-H, CF3, OCF3, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei bedeuten:
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann.

Besonders bevorzugt betrifft die Erfindung auch ein Verfahren zu Herstellung der Verbindungen der Formel IV worin bedeuten:
W = CH;
o = 1;
R¹ = H;
R² = H, CH₃, OCH₃, Br oder Cl;
R³ = H, CH₃, OCH₃, Br oder Cl;
R⁴ = CH₃, CH₂CH₃ oder CH(CH₃)₂;
R⁵ = H, CH₃, CH₂CH₃ oder CH(CH₃)₂;
R⁶ = H, CH₃, CH₂CH₃ oder CH(CH₃)₂;
X³ = Cl, CH₃SO₃ oder p-CH₃-C₆H₄-SO₃ und
n2 = 0 oder 1.

Die unsubstituierten oder substituierten Ammoniumionen in der Definition von R⁹ bedeuten vorzugsweise Triethylammonium.

Insbesondere betrifft die Erfindung ein Verfahren zur Herstellung von Verbindungen der Formel VIII, 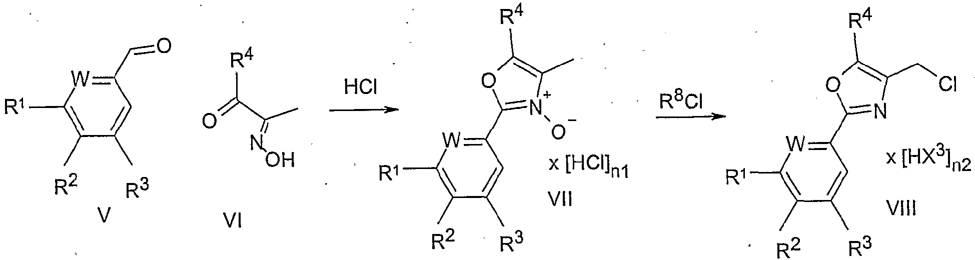 worin
R¹ = H,
R² = H oder CH₃ ,
R³ = H oder OCH₃ ,
R⁴ = CH₃ oder CH(CH₃)₂,
W = CH,
X³ = Cl oder CH₃SO₃ und
n2 = 0 oder 1 bedeuten.

Ganz besonders bevorzugt betrifft die Erfindung ein Verfahren, in dem das Reagens R⁸X² die Struktur
SOCl-Cl, SOBr-Br, CH₃SO₂-Cl oder p-CH₃-C₆H₄-SO₂-Cl aufweist.

Insbesondere betrifft die Erfindung ein Verfahren, in dem das Reagens R⁸X² entweder die Struktur SOCl-Cl (Formel IX) oder CH₃SO₂-Cl (Formel X) aufweist.

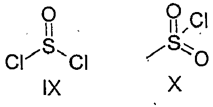

Das N-Oxid (Formel III) kann entweder isoliert oder direkt in Lösung weiter umgesetzt werden.

Wenn das N-Oxid (Formel III) oder das Oxazol (Formel IV) als Salz anfällt (n1 ≠0 oder n2 ≠0), dann kann es durch Behandlung mit einer Base, wie z. B. wässerigen Lösungen von Natriumhydroxid, Kaliumhydroxid, Natriumcarbonat, Kaliumcarbonat, Natriumhydrogencarbonat und Kaliumhydrogencarbonat, in die korrespondierende freie Base überführt werden.

Für die Reaktion zur Bildung der N-Oxide (Formel I + Formel II → Formel III) kommen als Reagens HX¹ Halogenwasserstoffe, Schwefelsäure und deren saures Salz, Phosphorsäure und deren saure Salze, Trifluoressigsäure, Trichloressigsäure, Trifluormethansulfonsäure, Methansulfonsäure, Benzolsulfonsäure, p-Toluolsulfonsäure, Ameisensäure sowie HMSO₄, H₂MPO₄, HM₂PO₄ mit M = Na, K in Betracht, wobei Halogenwasserstoffe bevorzugt sind. In einer besonders bevorzugten Ausführungsform wird man Chlorwasserstoff wählen. Im Fall von Schwefelsäure können Hydrogensulfate (n1 = 1) oder Sulfate (n1 = ½) entstehen, im Fall von Phosphorsäure können Dihydrogenphosphate (n1 = 1), Hydrogenphosphate (n1 = ½) oder Phosphate (n1 = 1/3) entstehen.

Das Reagens HX¹ kann in stöchiometrischen Mengen, bezogen auf das *α*-Ketoxim (Formel II), bis zu einem hohen Überschuss eingesetzt werden. Ein bevorzugter Arbeitsbereich ist der Einsatz von stöchiometrischen Mengen bis zu einem 7-fachen Überschuss. Besonders bevorzugt ist der 1 - 6-fache Überschuss.

Für die Reaktion zur Bildung der N-Oxide (Formel I + Formel II → Formel III) können als Lösungsmittel protische polare wie Carbonsäuren, aprotische dipolare wie Sulfoxide, Nitrile oder Ether bzw Polyether, aprotische polare wie halogenierte aromatische und aliphatische Kohlenwasserstoffe oder aprotische unpolare wie aromatische und aliphatische Kohlenwasserstoffe oder ein Gemisch aus den Lösungsmittelgruppen eingesetzt werden. So kommen z. B. Ameisensäure, Eisessig, Propionsäure, N,N-Dimethylformamid, N,N-Dimethylacetamid, N-Methylpyrrolidon, Dimethysulfoxid, Tetrahydrofuran, Diethylether, Diisopropylether, tert.-Butylmethylether, Ethylenglykoldimethylether und höhere Homolge oder Dichlormethan und Chlorbenzol oder Toluol, Cyclohexan und n-Heptan jeweils alleine oder im Gemisch in Betracht. In einer bevorzugten Form führt man die Reaktion in Eisessig, im Gemisch aus Eisessig und Ethylenglykoldimethylether oder im Gemisch aus Eisessig und Toluol durch.

Die Reaktionstemperaturen zur Bildung der N-Oxide (Formel I + Formel II → Formel III) lassen sich in einem weiten Bereich variieren und hängen unter anderem von den Löslichkeitseigenschaften der umzusetzenden Aldehyde (Formel I) und α-Ketoxime (Formel II) ab. So sind grundsätzlich Reaktionstemperaturen von minus 20°C bis 150°C möglich, wobei im allgemeinen Reaktionstemperaturen von minus 10°C bis 90°C zu bevorzugen sind. In einer besonders bevorzugten Durchführungsform wird man Reaktionstemperaturen von 0°C bis 60°C wählen.

Die Bildung der N-Oxide (Formel I + Formel II → Formel III) kann entweder im geschlossenen System unter Überdruck oder auch unter Normaldruck im offenen System durchgeführt werden, das heißt beispielsweise durch Einleiten eines Halogenwasserstoffgases in das zur Atmosphäre hin offene System oder durch Verwendung eines Halogenwasserstoffgases in einem organischen Lösungsmittel.

Ist unter den Resten R¹ bis R⁶ eine weitere Funktion, wie COOR⁹, die mit aktivierten Säurederivaten reagieren kann, so kann das Produkt als Säurederivat COX² oder nach vorheriger Verseifung nach im Prinzip bekannten Verfahren durch saure oder alkalische Hydrolyse als freie Säure COOH erhalten werden.

Das Reagens R⁸X² kann in stöchiometrischen Mengen, bezogen auf das intermediäre N-Oxid (Formel III), bis zu einem hohen Überschuss eingesetzt werden. Ein bevorzugter Arbeitsbereich ist der Einsatz von stöchiometrischen Mengen bis zu einem 5-fachen Überschuss. Besonders bevorzugt ist der 1 - 4-fache Überschuss. Dabei wird der Teil X² (von R⁸X²) in Formel IV kovalent gebunden eingebaut und R⁸ durch Hydrolyse in HX³ umgewandelt.

Für die Reaktion zur Bildung der Halogenmethyloxazole (Formel III → Formel IV) können als Lösungsmittel aprotische dipolare wie Amide, Sulfoxide, Nitrile oder Ether bzw Polyether, aprotische polare wie halogenierte aromatische und aliphatische Kohlenwasserstoffe oder aprotische unpolare wie aromatische und aliphatische Kohlenwasserstoffe oder ein Gemisch aus den Lösungsmittelgruppen eingesetzt werden. So kommen z. B. N,N-Dimethylformamid, N,N-Dimethylacetamid, N-Methylpyrrolidon, Dimethysulfoxid, Tetrahydrofuran, Diethylether, Diisopropylether, tert.-Butylmethylether, Ethylenglykoldimethylether und höhere Homologe oder Dichlor-methan und Chlorbenzol oder Toluol, Cyclohexan und n-Heptan jeweils alleine oder im Gemisch in Betracht. In einer bevorzugten Form führt man die Reaktion in Dichlormethan oder Toluol durch. Die Reaktion kann auch lösungsmittelfrei in einem Überschuss der Reagentien Thionylchlorid oder Methansulfonsäurechlorid durchgeführt werden.

Die Reaktionstemperaturen zur Bildung der Halogenmethyloxazole (Formel III → Formel IV) lassen sich in einem weiten Bereich variieren und hängen unter anderem von den Löslichkeitseigenschaften der umzusetzenden Aldehyde und α-Ketoxime ab. So sind grundsätzlich Reaktionstemperaturen von minus 20°C bis 150°C möglich, wobei im allgemeinen Reaktionstemperaturen von 20°C bis 120°C zu bevorzugen sind. In einer besonders bevorzugten Durchführungsform wird man Reaktionstemperaturen von 20°C bis 80°C wählen.

Halogen steht für Fluor, Chlor, Brom, Iod, bevorzugt für Fluor, Chlor, Brom, besonders bevorzugt für Chlor oder Brom und ganz besonders bevorzugt für Chlor.

Unter einem Alkylrest wird eine geradkettige oder verzweigte Kohlenwasserstoffkette mit einem bis sechs Kohlenstoffen verstanden, wie z.B. Methyl, Ethyl, Propyl, Butyl, Pentyl, Hexyl, Isopropyl, Isobutyl, Neopentyl, tert.-Butyl.
Die Alkylreste können ein-, zwei- oder dreifach mit geeigneten Gruppen substituiert sein, wie z.B.: F, Cl, Br, I, CF3, N02, N3, CN, COOH, COO(C1-C6)-Alkyl, CONH2, CONH(C1-C6)-Alkyl, CON[(C1-C6)-Alkyl]2, (C3-C8)-Cycloalkyl, (C2-C6)-Alkenyl, (C2-C6)-Alkinyl, (C6-C10)-Aryl.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein, zwei- oder dreifach mit geeigneten Gruppen substituiert sein, wie z.B.: F, Cl, Br, I, CF3, N02, SF5, N3, CN, COOH, COO(C1-C6)-Alkyl, CONH2, CONH(C1-C6)-Alkyl, CON[(C1-C6)Alkyl]2, (C3-C8)-Cycloalkyl, (C1-C10)-Alkyl, (C2-C6)-Alkenyl, (C2-C6)-Alkinyl, O-(C1-C6)-Alkyl, O-CO-(C1-C6)-Alkyl, O-CO-(C6-C10)-Aryl.

Unter einem Cycloalkylrest wird ein einen oder mehrere Ringe enthaltendes drei bis achtgliedriges Ringsystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylreste können ein- zwei- oder dreifach mit geeigneten Gruppen substituiert sein, wie z.B.: F, Cl, Br, I, CF3, N02, N3, CN, COOH, COO(C1-C6)-Alkyl, CONH2, CONH(C1-C6)-Alkyl, CON[(C1-C6)Alkyl]2, (C3-C8)-Cycloalkyl, (C1-C10)-Alkyl, (C2-C6)-Alkenyl, (C2-C6)-Alkinyl, O-(C1-C6)-Alkyl, O-CO-(C1-C6)-Alkyl, O-CO-(C6-C10)-Aryl.

Unter einem Heteroarylrest wird ein C5-C6-Heterocyclus verstanden, der 1- bis 4 Heteroatome aus der Reihe O, N, S enthalten kann. Beispielsweise seien genannt: Furan, Thiophen, Pyrrol, Pyridin, Pyrazin, Pyrimidin, Pyridazin, Oxazol, Isoazol, Thiazol, Isothiazol, Furazan, Tetrazol.

Die erfindungsgemäßen Verbindungen der Formel IV können beispielsweise entsprechend der DE 102004040736.3 weiter zu pharmazeutisch wirksamen Stoffen, den PPAR Agonisten umgesetzt werden.

### Beispiel 1

### 2-(3-Methoxy-phenyl)-4,5-dimethyl-oxazol-3-oxid (Formel XI)

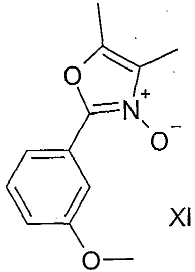

15,2 g (0,150 mol) 2,3-Butandionmonoxim wurden vorgelegt und 260 mL Toluol, 22,1 g (0,157 mol) 3-Methoxybenzaldehyd und 70 mL (73,4 g, 1,224 mol) Eisessig unter Rühren zugegeben. 27,3 g (0,749 mol) Chlorwasserstoffgas wurden unter Kühlung eingeleitet, so dass die Innentemperatur < 22°C betrug. Anschliessend wurde bis zu 16 h nachgerührt. Unter Rühren wurde die Reaktionsmischung zu 600 mL Wasser gegeben (exotherme Reaktion). Der pH-Wert wurde auf 10,6 gestellt, dazu wurden 172 mL (1,930 mol) 33 % wässrige Natronlauge benötigt, die Innentemperatur wurde durch externe Kühlung < 32°C gehalten. Es bildeten sich zwei Phasen, die getrennt wurden. Die wässerige Phase wurde 2 mal mit je 100 mL Toluol extrahiert und anschliessend verworfen. Die vereinigten organischen Phasen wurden im Vakuum, unter Abdestillieren von 50 mL, konzentriert. Die so erhaltene toluolische Lösung (420 mL) wurde direkt zur Synthese von 4-Chlormethyl-2-(3-methoxy-phenyl)-5-methyl-oxazol Hydrochlorid eingesetzt.
Ausbeute: 32,9 g (100 %) 2-(3-Methoxy-phenyl)-4,5-dimethyl-oxazol-3-oxid, nicht isoliert, Annahme zur Berechnung der Folgestufe.

Die folgenden Daten wurden an der Reinsubstanz gemessen, die nach dem kompletten Abdestillieren des Lösungsmittels der organischen Phasen erhalten wurde. Schmelzpunkt: 114°C
¹H-NMR (CDCl₃, 400 MHz) *δ* (ppm) = 2,20 (s, 3 H); 2,35 (s, 3H); 3,87 (s, 3 H); 6,98 (m, 1 H); 7,38 (m, 1 H); 7,88 (m, 3 H); 8,26 (m, 1 H).

### Beispiel 2

### 4-Chlormethyl-2-(3-methoxy-phenyl)-5-methyl-oxazol Hydrochlorid (Formel XII)

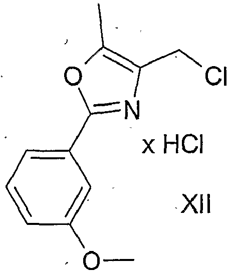

Die gesamte toluolische Lösung aus Beispiel 1 (420 mL) wurde bei < 60°C tropfenweise mit 54,2 g (0,456 mol) Thionylchlorid versetzt und bei < 60°C bis zu 22 h gerührt. Anschliessend wurde durch Abdestillieren von 229 mL konzentriert. Die Suspension wurde auf < 20°C gekühlt, und das Produkt durch Absaugen isoliert, 3 mal mit je 20 ml Toluol gewaschen und im Vakuum bei erhöhter Temperatur getrocknet.
Ausbeute: 23,2 g (56 %) 4-Chlormethyl-2-(3-methoxy-phenyl)-5-methyl-oxazol Hydrochlorid
Schmelzpunkt: 117°C
¹H-NMR (CDCl₃, 400 MHz) *δ* (ppm) = 2,58 (s, 3 H); 3,92 (s, 3 H); 4,78 (s, 2 H); 7,15 (m, 1 H); 7,42 (m, 1 H); 7,79 (m, 1 H); 8,04 (m, 1 H).

### Beispiel 3

### 4-Chlormethyl-2-(3-methoxy-phenyl)-5-methyl-oxazol (Formel XIII)

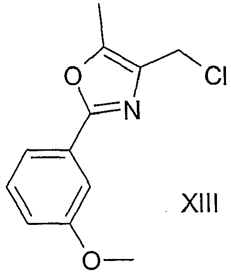

10,1 g (0,037 mol) 4-Chlormethyl-2-(3-methoxy-phenyl)-5-methyl-oxazol Hydrochlorid wurden in 100 mL Wasser und 75 mL Dichlormethan suspendiert. Unter Rühren wurde in der Wasserphase mit 45 mL (0,023 mol) wässeriger Natronlauge ein pH-Wert von 12 eingestellt. Anschliessend wurden die Phasen getrennt, und die wässerige Phase verworfen. Die organische Phase wurde im Vakuum komplett eindestilliert. Das zurückbleibende Öl kristallisierte nach der Zugabe von Impfkristallen durch.
Ausbeute: 8,0 g (92 %) 4-Chlormethyl-2-(3-methoxy-phenyl)-5-methyl-oxazol Schmelzpunkt: 46 - 50°C
¹H-NMR (CDCl₃, 400 MHz) *δ* (ppm) = 2,43 (s, 3 H); 3,88 (s, 3 H); 4,56 (s, 2 H); 6,99 (m, 1 H); 7,35 (m, 1 H); 7,54 (m, 1 H); 7,60 (m, 1 H).

### Beispiel 4

### 4,5-Dimethyl-2-p-tolyl-oxazol-3-oxid Hydrochlorid (Formel XIV)

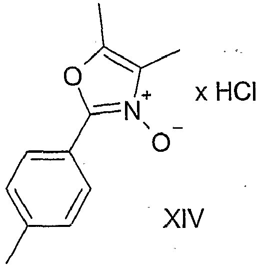

100 g (979 mmol) Butan-2,3-dionmonoxim wurden vorgelegt und in 500 mL Essigsäure gelöst. 120 g (979 mmol) 4-Methylbenzaldehyd wurden zugegeben. 100 g (2,74 mol) Chlorwasserstoffgas wurden so eingeleitet, dass eine Innentemperatur von 40°C nicht überschritten wurde. Anschließend wurde 2-3 Stunden bei 35-40°C nachgerührt. Unter intensiver Kühlung wurden 2 L tert.-Butylmethylether zugegeben. Das Reaktionsgemisch wurde 1 Stunde bei 10°C gerührt. Das Produkt wurde durch Absaugen isoliert, mit tert.-Butylmethylether nachgewaschen und im Vakuum bei erhöhter Temperatur getrocknet.
Ausbeute: 213 g (91 %) 4,5-Dimethyl-2-p-tolyl-oxazol-3-oxid Hydrochlorid
Schmelzpunkt: 101°C
¹H-NMR (DMSO-D₆, 500 MHz) *δ* (ppm) = 10,30 (s_{br}, 1H), 8,17 (d, J = 8,3 Hz; 2H), 7,47 (d, J = 8,3 Hz; 2H), 2,44 (s, 3H), 2,42 (s, 3H)

### Beispiel 5

### 4-Chlormethyl-5-methyl-2-p-tolyl-oxazol (Formel XV)

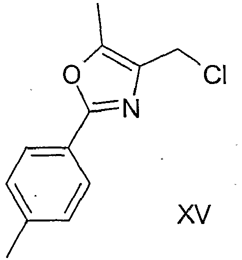

32,8 g (137 mmol) 4,5-Dimethyl-2-p-tolyl-oxazol-3-oxid Hydrochlorid wurden in 165 mL Dichlormethan suspendiert. 17,5 g (151 mmol) Methansulfonylchlorid wurden zugegeben. Die Rektion wurde bis zum vollständigen Umsatz (HPLC) am Rückfluss gerührt. Anschließend wurden 200 mL Ethylenglykoldimethylether zugegeben, und das Dichlormethan im Vakuum abdestilliert. Das Reaktionsgemisch wurde auf 15°C gekühlt und 250 mL Wasser wurden zugegeben. Das Gemisch wurde 1 Stunde bei 15°C gerührt. Das ausgefallene Produkt wurde durch Absaugen isoliert, mit Wasser nachgewaschen und im Vakuum bei erhöhter Temperatur getrocknet.
Ausbeute: 27,6 g (91 %) 4-Chlormethyl-5-methyl-2-p-tolyl-oxazol
Schmelzpunkt: 95°C
¹H-NMR (DMSO-D₆, 500 MHz) *δ* (ppm) = 7,82 (d, J = 8,1 Hz, 2H), 7,33 (d, J = 8,1 Hz, 2H), 4,74 (s, 2H), 2,43 (s, 3H), 2,37 (s, 3H)

### Beispiel 6

### 4-Methyl-pentane-2,3-dion-2-oxim (Formel XVI)

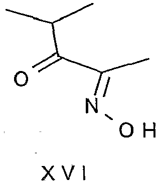

100 g (948 mmol) 2-Methyl-pentan-3-on wurden in 400 mL tert.-Butylmethylether gelöst. 50 g (274 mmol) Lösung von Chlorwasserstoff in Ethylenglykoldimethylether (20 %ig) wurden zugegeben. Anschließend wurde eine Lösung von 117 g (949 mmol) iso-Amylnitrit in 150 mL tert.-Butylmethylether innerhalb von 60 Minuten zugetropft. Das Lösungsmittel wurde im Vakuum vollständig entfernt. Der Rückstand wurde in 300 mL n-Heptan aufgenommen und erneut im Vakuum eingeengt. Nach Zugabe von 200 mL n-Heptan wurde mit 522 mL Natronlauge (2 molar) extrahiert. Nach Phasentrennung wurde die wässerige Phase mit n-Heptan gewaschen. Die wässerige Phase wurde durch Zugabe von konz. Salzsäure angesäuert. Das Produkt wurde durch Absaugen isoliert, mit Wasser nachgewaschen und im Vakuum bei erhöhter Temperatur getrocknet.
Ausbeute: 61,1 g (50%)4-Methyl-pentane-2,3-dion-2-oxim
Schmelzpunkt: 94°C
¹H-NMR (DMSO-D₆, 500 MHz) *δ* (ppm) = 12,3 (s, 1 H), 3,54 (sept, J = 6,9 Hz, 1 H), 1,82 (2, 3H), 1,02 (s, 3H), 1,01 (s, 3H).

### Beispiel 7

### 5- Isopropyl-2-(3-methoxy-phenyl)-4-methyl-oxazol 3-oxid (Formel XVII)

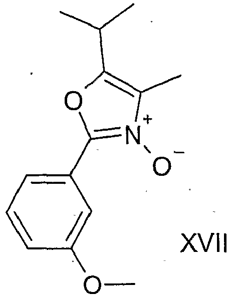

Zu einer Lösung von 18,0 g (137 mmol) 4-Methyl-pentane-2,3-dion-2-oxim in 30 g (99 mmol) Lösung von Chlorwasserstoff in Essigsäure (12 %ig) und 30 g (164 mmol) Lösung von Chlorwasserstoff in Ethylenglykoldimethylether (20 %ig) wurden 19,0 g (137 mmol) 3-Methoxybenzaldehyd gegeben. Die Reaktion wurde 3 Stunden bei 50-55°C und 60 Stunden bei Zimmertemperatur gerührt. Anschließend wurden 500 mL Wasser und 300 mL tert.-Butylmethylether zugegeben, bevor durch Zugabe von Natriumhydrogencarbonat ein pH-Wert von 3-4 eingestellt wurde. Nach Phasentrennung wurde die wässerige Phase zweimal mit je 100 mL tert.-Butylmethylether extrahiert. Die vereinigten organischen Phasen wurden mit 4 x 100 mL Wasser gewaschen und im Vakuum vollständig eingeengt.
Ausbeute: 42,8 g (79 %ig) (100 %) 5- Isopropyl-2-(3-methoxy-phenyl)-4-methyl-oxazol 3-oxid
¹H-NMR DMSO-D₆, 500 MHz) *δ* (ppm) = 8,12 (m, 1H), 7,86 (d, J = 8,0 Hz, 1H), 7,48 (t, J = 8,0 Hz, 1 H), 7,06 (dd, J = 2,4, 8,0 Hz, 1 H), 3,82 (s, 3H), 3,16 (sept, J = 7,0 Hz, 1 H), 2,12 (s, 3H), 1,29 (d, J = 7,0 Hz, 3H).

### Beispiel 8

### 4-Chlormethyl-5-isopropyl-2-(3-methoxy-phenyl)-oxazol (Formel XVIII)

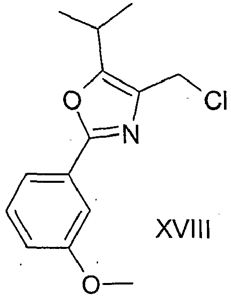

Zu einer Lösung von 135 g (435 mmol) 5- Isopropyl-2-(3-methoxy-phenyl)-4-methyl-oxazol 3-oxid in 500 mL Dichlormethan wurden bei einer Temperatur von 20°C 75 g (648 mmol) Methansulfonsäurechlorid gegeben. Die Reaktion wurde bis zum vollständigem Umsatz bei 40-45°C gerührt. Es wurden 500 mL tert.-Butylmethylether und 300 mL Wasser zugegeben. Durch Zugabe von 20 %iger Natronlauge wurde ein pH-Wert von 8 eingestellt. Nach Phasentrennung wurde die organische Phase mit 3 x 200 mL Wasser gewaschen. Die organische Phase wurde im Vakuum vollständig eingeengt.
Ausbeute: 132 g (87%ig) (99 %) 4-Chlormethyl-5-isopropyl-2-(3-methoxy-phenyl)-oxazol
¹H-NMR DMSO-D₆, 500 MHz) *δ* (ppm) = 7,55 (m, 1H), 7,45 (m, 2H), 7,10 (ddd, J = 0,9, 2,7, 5,6 Hz, 1 H), 4,77 (s, 2H), 3,85 (s, 3H), 3,33 (sept, 7,0 Hz, 1 H), 1,30 (d, J = 7,0 Hz, 6H).

## Patentansprüche

1. Verfahren zur Herstellung der Verbindungen der Formel IV, 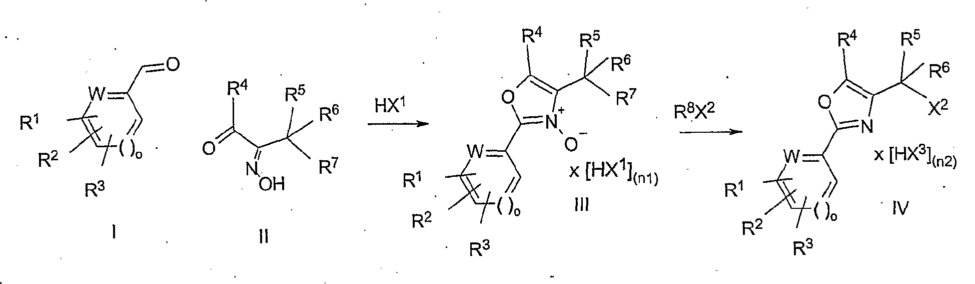 **dadurch gekennzeichnet, dass** man die aromatischen Aldehyde der Formel I mit den α-Ketoximen der Formel II 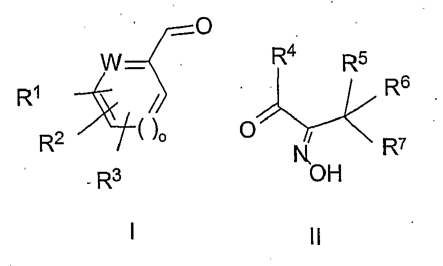 worin bedeuten:
R¹ H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹ , wobei Aryl unsubstituiert oder mono-, di- oder trisubstituiert ist durch F, Cl, Br, I, (C1-C4)-Alkyl, O-(C1-C4)-Alkyl oder CF₃;
wobei
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl;
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann;
R² H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind;
R³ H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind;
W CH, N, falls o = 1;
W O, S, NR12, falls o = 0;
o 0 oder 1;
R12 H, (C1-C6)-Alkyl, (C1-C6)-Alkylen-Phenyl, Phenyl;
R⁴ H, (C1-C8)-Alkyl, (C3-C8)-Cycloalkyl, (C1-C3)-Alkylen-(C3-C8)-Cycloalkyl, Phenyl, (C1-C3)-Alkylen-Phenyl, (C5-C6)-Heteroaryl, (C1-C3)-Alkylen-(C5-C6)-Heteroaryl oder (C1-C3)-Alkyl, das durch F ganz oder teilweise substituiert ist, oder COOR⁹, CONR(10)R(11);
wobei R⁹, R¹⁰ und R¹¹ wie oben definiert sind;
R⁵ und R⁶ unabhängig voneinander
H, (C1-C8)-Alkyl, F, Cl, Br, I, O-(C0-C8)-Alkylen-H, O-(C6-C10)-Aryl, O-(C1-C4)-Alkylen-(C6-C10)-Aryl, COOR⁹, CONR¹⁰R¹¹, SH oder NR¹⁰R¹¹,
wobei R⁹, R¹⁰, R¹¹ wie oben definiert sind;
oder
R⁵ und R⁶ gemeinsam
für (C4-C5)-Alkylen steht, wobei eine CH2-Gruppe durchO, S, NH, N-CH₃ oder N-Benzyl ersetzt sein kann;
R⁷ H oder (C1-C8)-Alkyl;
in Gegenwart von Säuren
in die N-Oxide der Formel III überführt, 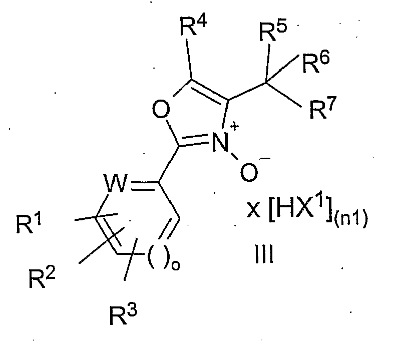 worin R¹, R², R³, R⁴, R⁵, R⁶, R⁷ und X¹ wie oben definiert sind und
n1 0, 1, ½ oder 1/3 bedeutet;
und diese anschließend
mit dem Reagens R⁸X² , welches bedeutet:
SOCl-Cl, SOBr-Br, CH₃SO₂-Cl, CF₃SO₂-Cl, C₆H₅SO₂-Cl, p-CH₃-C₆H₄-SO₂-Cl, CH₃SO₂-O₃SCH₃, CF₃SO₂-O₃SCF₃, C₆H₅SO₂-O₃SC₆H₅ oder p-CH₃-C₆H₄-SO₂-O₃S-C₆H₄-p-CH₃,
zu den Halogenmethyloxazolen der Formel IV umsetzt, 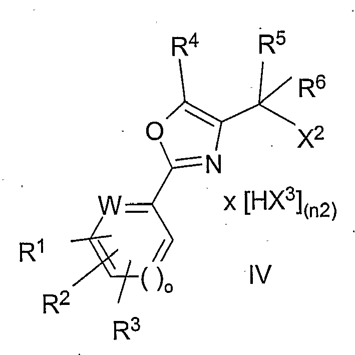 worin R¹, R², R³, R⁴, R⁵, R⁶ und X² wie oben definiert sind und
X3 Cl, Br, CH₃SO₃, CF₃SO₃, C₆H₅SO₃, oder p-CH₃-C₆H₄-SO₃ und
n2 0 oder 1 bedeuten.

2. Verfahren zur Herstellung der Verbindungen der Formel IV gemäß Anspruch 1, worin bedeuten:
W = CH und
o = 1.

3. Verfahren zu Herstellung der Verbindungen der Formel IV gemäß Anspruch 1 oder 2, worin bedeuten:
R¹ H;
R² H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei bedeuten:
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl;
R¹⁰ und R¹¹ unabhängig voneinander H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann;
R³ H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei R⁹, R¹⁰ und R¹¹ wie oben definiert sind.

4. Verfahren zu Herstellung der Verbindungen der Formel IV gemäß den Ansprüchen 1 bis 3, worin bedeuten:
R1 H;
R² H;
R³ H, (C1-C6)-Alkyl, F, Cl, Br, I, O-(C0-C8)-Alkylen-H, CF3, OCF3, SCF3, SF5, OCF2-CHF2, (C6-C10)-Aryl, O-(C6-C10)-Aryl, O-(C1-C4)-Alkylen-(C6-C10)-Aryl, N02, COOR⁹, CONR¹⁰R¹¹, SH, oder NR¹⁰R¹¹, wobei Aryl unsubstituiert oder mono-, di- oder trisubstituiert ist durch F, Cl, Br, I, (C1-C4)-Alkyl, O-(C1-C4)-Alkyl oder CF₃;
wobei
R⁹ H, Li, Na, K, 1/2Mg, 1/2Ca, unsubstituierte oder mit (C1-C4)-Alkyl mono-, di- oder trisubstituierte Ammoniumionen, oder (C1-C8)-Alkyl
R¹⁰ und R¹¹ unabhängig voneinander
H, (C1-C5)-Alkyl, Phenyl oder CH2-Phenyl,
wobei Phenyl unsubstituiert oder mono-, di- oder trisubstituiert ist mit F, Cl, Br, I, (C1-C4)-Alkyl, O-(C1-C4)-Alkyl oder CF₃;
oder
R¹⁰ und R¹¹ gemeinsam für (C4-C5)-Alkylen steht, wobei eine CH2-Gruppe durch O, S, NH, N-CH₃ oder N-Benzyl ersetzt sein kann.

5. Verfahren zu Herstellung der Verbindungen der Formel IV gemäß den Ansprüchen 1 bis 4, worin bedeuten:
W = CH;
o = 1;
R¹ = H;
R² = H, CH₃, OCH₃, Br oder Cl;
R³ = H, CH₃, OCH₃, Br oder Cl;
R⁴ = CH₃, CH₂CH₃ oder CH(CH₃)₂;
R⁵ = H, CH₃, CH₂CH₃ oder CH(CH₃)₂;
R⁶ = H, CH₃, CH₂CH₃ oder CH(CH₃)₂;
X³ = Cl, CH₃SO₃ oder p-CH₃-C₆H₄-SO₃ und
n2 = 0 oder 1.

6. *(geändert)* Verfahren zur Herstellung von Verbindungen der Formel IV entsprechend Anspruch 1, wobei die Verbindungen der Formel IV bevorzugt durch die Formel VIII definiert sind 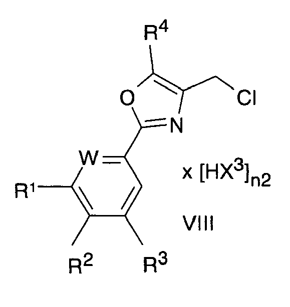 worin
R¹ = H,
R² = H oder CH₃,
R3 = H oder OCH₃,
R⁴ = CH₃ oder CH(CH₃)₂,
W = CH,
X³ = Cl oder CH₃SO₃ und
n2 = 0 oder 1 bedeuten.

7. Verfahren zur Herstellung der Verbindungen der Formeln IV oder VIII gemäß den Ansprüchen 1 bis 6, wobei die Reaktionstemperatur zur Bildung der N-Oxide (Formel III) aus Verbindungen der Formeln I und II zwischen -20°C und +150°C liegt.

8. Verfahren zur Herstellung der Verbindungen der Formeln IV oder VIII gemäß den Ansprüchen 1 bis 7, wobei die Reaktion zur Bildung der N-Oxide (Formel III) in protischen, polaren, aprotischen unpolaren oder Gemischen der genannten Lösungsmittel erfolgt.

9. Verfahren zur Herstellung der Verbindungen der Formeln IV oder VIII gemäß den Ansprüchen 1 bis 8, wobei die Reaktionstemperatur zur Bildung der Halogenmethyloxazole (Formel IV) aus Verbindungen der Formel III -20°C bis +150°C liegt.

10. Verfahren zur Herstellung der Verbindungen der Formeln IV oder VIII gemäß den Ansprüchen 1 bis 9, wobei die Reaktion zur Bildung der Halogenmethyloxazole (Formel IV) in aprotischen dipolaren, aprotischen polaren, aprotischen unpolaren oder Gemischen der genannten Lösungsmittel erfolgt.

11. Verfahren zur Herstellung der Verbindungen der Formeln IV oder VIII gemäß den Ansprüchen 1 bis 10, wobei die Verbindung der Formel R⁸X² in einem 1- bis 4-fachen Überschuss bezogen auf das N-Oxid (Formel III) eingesetzt wird.

12. Verwendung einer oder mehrerer der Verbindungen der Formel I, II, III oder IV in einem Verfahren zur Herstellung der Verbindungen der Formeln IV oder VIII gemäß den Ansprüchen 1 bis 11.

## Claims

1. A process for preparing compounds of the formula IV, 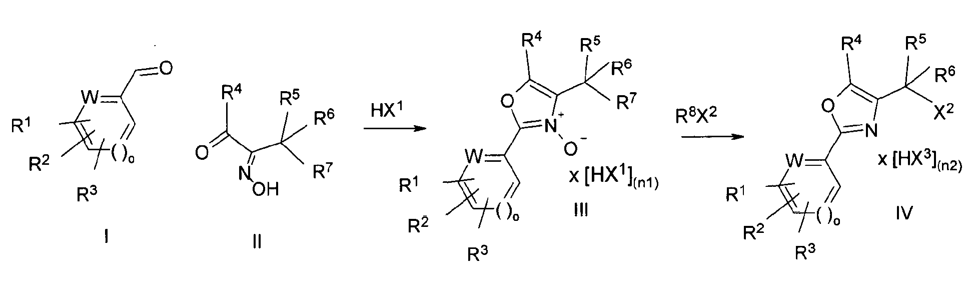 which comprises converting the aromatic aldehydes of the formula I using the α-ketoximes of the formula II 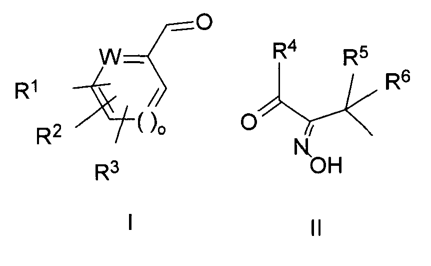 in which:
R¹ is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, 0-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where
R⁹ is H, Li, Na, K, 1/2Mg, 1/2Ca, ammonium ions which are unsubstituted or mono-, di- or trisubstituted by (C1-C4)-alkyl, or is (C1-C8)-alkyl,
R¹⁰ and R¹¹ are each independently H, (C1-C5)-alkyl, phenyl or CH₂-phenyl,
where phenyl is unsubstituted or mono-, di- or trisubstituted by F, Cl, Br, I, (C1-C4)-alkyl, 0-(C1-C4)-alkyl or CF₃;
or
R¹⁰ and R¹¹ together are (C4-C5)-alkylene, in which one CH₂ group may be replaced by 0, S, NH, N-CH₃ or N-benzyl;
R² is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, O-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where R⁹, R¹⁰ and R¹¹ are each as defined above;
R³ is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, O-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where R⁹, R¹⁰ and R¹¹ are each as defined above;
W is CH, N, if o = 1;
W is 0, S, NR12, if o = 0;
o is 0 or 1;
R12 is H, (C1-C6)-alkyl, (C1-C6)-alkylenephenyl, phenyl;
R⁴ is H, (C1-C8)-alkyl, (C3-C8)-cycloalkyl, (C1-C3)-alkylene-(C3-C8)-cycloalkyl, phenyl, (C1-C3)-alkylenephenyl, (C5-C6)-heteroaryl, (C1-C3)-alkylene-(C5-C6)-heteroaryl or (C1-C3)-alkyl which is fully or partly substituted by F, or COOR⁹, CONR(10)R(11);
where R⁹, R¹⁰ and R¹¹ are each as defined above;
R⁵ and R⁶ are each independently
H, (C1-C8)-alkyl, F, Cl, Br, I, O-(C0-C8)-alkylene-H, O-(C6-C10)-aryl, O-(C1-C4)-alkylene-(C6-C10)-aryl, COOR⁹, CONR¹⁰R¹¹, SH or NR¹⁰R¹¹,
where R⁹, R¹⁰, R¹¹ are each as defined above;
or
R⁵ and R⁶ together
are (C4-C5)-alkylene, in which one CH₂ group may be replaced by 0, S, NH, N-CH₃ or N-benzyl;
R⁷ is H or (C1-C8)-alkyl;
in the presence of acids
to the N-oxides of the formula III 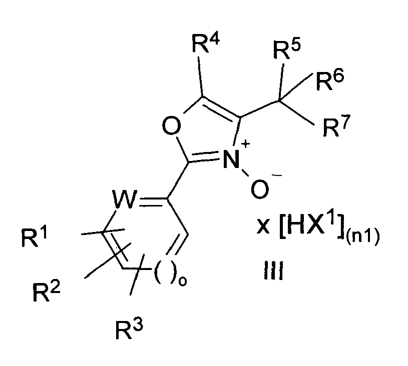 in which R¹, R², R³, R⁴, R⁵, R⁶, R⁷ and X¹ are each as defined above and
n1 is 0, 1, ½ or 1/3;
and the latter is subsequently reacted
with the reagent R⁸X² which means:
SOCl-Cl, SOBr-Br, CH₃SO₂-Cl, CF₃SO₂-Cl, C₆H₅SO₂-Cl, p-CH₃-C₆H₄-SO₂-Cl, CH₃SO₂-O₃SCH₃, CF₃SO₂-O₃SCF₃, C₆H₅SO₂-O₃SC₆H₅ or p-CH₃-C₆H₄-SO₂-O₃S-C₆H₄-p-CH₃,
to give the halomethyloxazoles of the formula IV 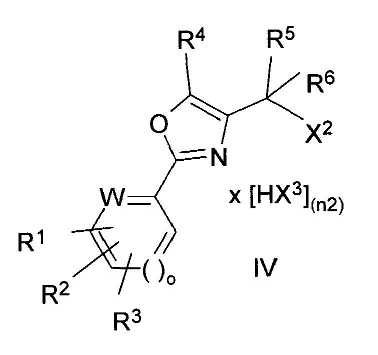 in which R¹, R², R³, R⁴, R⁵, R⁶ and X² are each as defined above and
X³ is Cl, Br, CH₃SO₃, CF₃SO₃, C₆H₅SO₃ or p-CH₃-C₆H₄-SO₃ and
n2 is 0 or 1.

2. The process for preparing the compounds of the formula IV as claimed in claim 1, in which:
W = CH and
o = 1.

3. The process for preparing the compounds of the formula IV as claimed in claim 1 or 2, in which:
R1 is H;
R² is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, 0-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where
R⁹ is H, Li, Na, K, 1/2Mg, 1/2Ca, ammonium ions which are unsubstituted or mono-, di- or trisubstituted by (C1-C4)-alkyl, or is (C1-C8)-alkyl,
R¹⁰ and R¹¹ are each independently H, (C1-C5)-alkyl, phenyl or CH₂-phenyl,
where phenyl is unsubstituted or mono-, di- or trisubstituted by F, Cl, Br, I, (C1-C4)-alkyl, O-(C1-C4)-alkyl or CF₃;
or
R¹⁰ and R¹¹ together are (C4-C5)-alkylene, in which one CH₂ group may be replaced by 0, S, NH, N-CH₃ or N-benzyl;
R³ is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, O-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where R⁹, R¹⁰ and R¹¹ are each as defined above.

4. The process for preparing the compounds of the formula IV as claimed in claims 1 to 3, in which:
R1 is H;
R² is H;
R³ is H, (C1-C6)-alkyl, F, Cl, Br, I, O-(C0-C8)-alkylene-H, CF3, OCF3, SCF3, SF5, OCF2-CHF2, (C6-C10)-aryl, O-(C6-C10)-aryl, O-(C1-C4)-alkylene-(C6-C10)-aryl, N02, COOR⁹, CONR¹⁰R¹¹, SH, or NR¹⁰R¹¹, where aryl is unsubstituted or mono-, di- or trisubstituted by F, Cl, Br, I, (C1-C4)-alkyl, O-(C1-C4)-alkyl or CF₃;
where
R⁹ is H, Li, Na, K, 1/2Mg, 1/2Ca, ammonium ions which are unsubstituted or mono-, di- or trisubstituted by (C1-C4)-alkyl, or is (C1-C8)-alkyl,
R¹⁰ and R¹¹ are each independently
H, (C1-C5)-alkyl, phenyl or CH₂-phenyl, where phenyl is unsubstituted or mono-, di- or trisubstituted by F, Cl, Br, I, (C1-C4)-alkyl, O-(C1-C4)-alkyl or CF₃;
or
R¹⁰ and R¹¹ together are (C4-C5)-alkylene, in which one CH₂ group may be replaced by 0, S, NH, N-CH₃ or N-benzyl.

5. The process for preparing the compounds of the formula IV as claimed in claims 1 to 4, in which:
W = CH;
o = 1;
R¹ = H;
R² = H, CH₃, OCH₃, Br or Cl;
R³ = H, CH₃, OCH₃, Br or Cl;
R⁴ = CH₃, CH₂CH₃ or CH(CH₃)₂;
R⁵ = H, CH₃, CH₂CH₃ or CH(CH₃)₂;
R⁶ = H, CH₃, CH₂CH₃ or CH(CH₃)₂;
X³ = Cl, CH₃SO₃ or p-CH₃-C₆H₄-SO₃ and
n2 = 0 or 1.

6. The process for preparing compounds of the formula IV according to claim 1, the compounds of the formula IV preferably being defined by the formula VIII 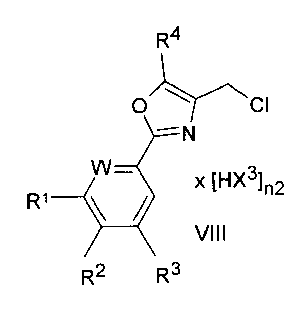 in which
R¹ = H,
R² = H or CH₃,
R³ = H or OCH₃,
R⁴ = CH₃ or CH(CH₃)₂,
W = CH,
X³ = Cl or CH₃SO₃ and
n2 = 0 or 1.

7. The process for preparing the compounds of the formulae IV or VIII as claimed in claims 1 to 6, wherein the reaction temperature for the formation of the N-oxides (formula III) from compounds of the formulae I and II is between -20°C and +150°C.

8. The process for preparing the compounds of the formulae IV or VIII as claimed in claims 1 to 7, wherein the reaction for the formation of the N-oxides (formula III) is effected in protic, polar, aprotic nonpolar solvents or mixtures of the solvents mentioned.

9. The process for preparing the compounds of the formulae IV or VIII as claimed in claims 1 to 8, wherein the reaction temperature for the formation of the halomethyloxazoles (formula IV) from compounds of the formula III is from -20°C to +150°C.

10. The process for preparing the compounds of the formula IV or VIII as claimed in claims 1 to 9, wherein the reaction for the formation of the halomethyloxazoles (formula IV) is effected in aprotic dipolar, aprotic polar, aprotic nonpolar solvents or mixtures of the solvents mentioned.

11. The process for preparing the compounds of the formulae IV or VIII as claimed in claims 1 to 10, wherein the compound of the formula R⁸X² is used in a from 1 to 4-fold excess based on the N-oxide (formula III).

12. The use of one or more of the compounds of the formula I, II, III or IV in a process for preparing the compounds of the formula IV or VIII as claimed in claims 1 to 11.

## Revendications

1. Procédé pour la préparation de composés de formule IV, 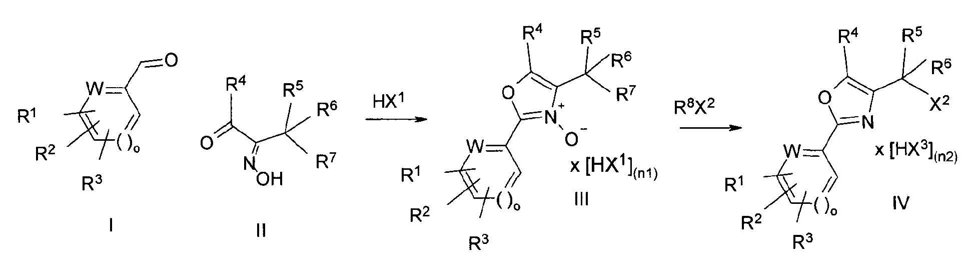 comprenant la conversion des aldéhydes aromatiques de formule I, à l'aide des α-cétoximes de formule II 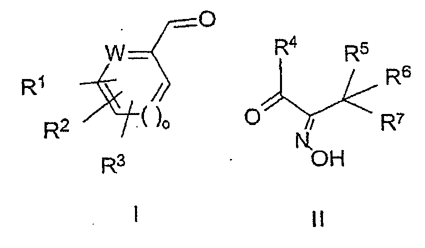 formules dans lesquelles :
R¹ est H, un groupe alkyle en C₁-C₆, F, Cl, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR⁹, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où
R⁹ est H, Li, Na, K, 1/2Mg, 1/2Ca, des ions ammonium non substitués ou mono-, di- ou trisubstitués par alkyle en C₁-C₄, ou est un groupe alkyle en C₁-C₈,
R¹⁰ et R¹¹ représentent chacun indépendamment H, un groupe alkyle en C₁-C₅, phényle ou CH₂-phényle,
le radical phényle étant non substitué ou mono-, di- ou trisubstitué par F, Cl, Br, I, alkyle en C₁-C₄, O-alkyle en C₁-C₄ ou CF₃ ;
ou
R¹⁰ et R¹¹ forment ensemble un groupe alkylène en C₄-C₅, dans lequel un groupe CH₂ peut être remplacé par O, S, NH, N-CH₃ ou N-benzyle ;
R² est H, un groupe alkyle en C₁-C₆, F, CI, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR9, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où R⁹, R¹⁰ et R¹¹ sont chacun tels que définis plus haut ;
R³ est H, un groupe alkyle en C₁-C₆, F, Cl, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR9, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où R⁹, R¹⁰ et R¹¹ sont chacun tels que définis plus haut ;
W est CH, N, si o = 1 ;
W est O, S, NR12, si o = 0 ;
o est 0 ou 1 ;
R12 est H, un groupe alkyle en C₁-C₆, alkylène(C₁-C₆)-phényle, phényle ;
R⁴ est H, un groupe alkyle en C₁-C₈, cycloalkyle en C₃-C₈, alkylène(C₁-C₃)-cycloalkyle(C₃-C₈), phényle, alkylène(C₁-C₃)-phényle, hétéroaryle en C₅-C₆, alkylène(C₁-C₃)-hétéroaryle(C₅-C₆) ou alkyle en C₁-C₃ qui est partiellement ou totalement substitué par F, ou COOR⁹, CONR(10)R(11) ;
où R⁹, R¹⁰ et R¹¹ sont tels que définis plus haut ;
R⁵ et R⁶ représentent chacun indépendamment
H, un groupe alkyle en C₁-C₈, F, Cl, Br, I, O-alkylène(C₀-C₈)-H, O-aryle en C₆-C₁₀, O-alkylène(C₁-C₄)-aryle(C₆-C₁₀), COOR⁹, CONR¹⁰R¹¹, SH ou NR¹⁰R¹¹,
où R⁹, R¹⁰, R¹¹ sont chacun tels que définis plus haut ;
ou
R⁵ et R⁶ forment ensemble
un groupement alkylène en C₄-C₅, dans lequel un groupe CH₂ peut être remplacé par O, S, NH, N-CH₃ ou N-benzyle ;
R⁷ est H ou un groupe alkyle en C₁-C₈ ;
en présence d'acides
en les N-oxydes de formule III 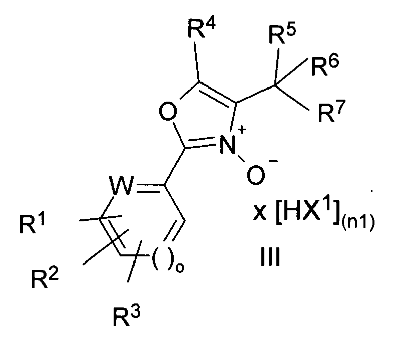 dans laquelle R¹, R², R³, R⁴, R⁵, R⁶, R⁷ et X¹ sont chacun tels que définis plus haut et
n1 est 0, 1, ½ ou 1/3 ;
et on fait ensuite réagir ces derniers
avec le réactif R⁸X² qui représente :
SOCI-CI, SOBr-Br, CH₃SO₂-Cl, CF₃SO₂-Cl, C₆H₅SO₂-Cl, p-CH₃-C₆H₄-SO₂-Cl, CH₃SO₂-O₃SCH₃, CF₃SO₂-O₃SCF₃, C₆H₅SO₂-O₃SC₆H₅ ou p-CH₃-C₆H₄-SO₂-O₃S-C₆H₄-p-CH₃,
pour obtenir les halogénométhyloxazoles de formule IV 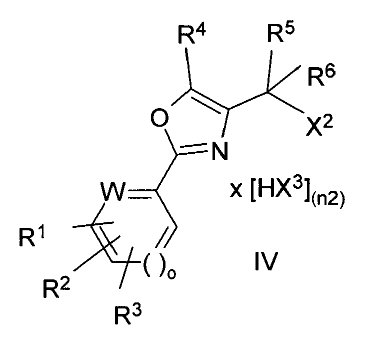 dans laquelle R¹, R², R³, R⁴, R⁵, R⁶ et X² sont chacun tels que définis plus haut et
X3 est Cl, Br, CH₃SO₃, CF₃SO₃, C₆H₅SO₃ ou p-CH₃-C₆H₄-SO₃ et
n2 est 0 ou 1.

2. Procédé pour la préparation des composés de formule IV selon la revendication 1, dans laquelle :
W = CH et
o = 1.

3. Procédé pour la préparation des composés de formule IV selon la revendication 1 ou 2, dans laquelle :
R1 est H;
R² est H, un groupe alkyle en C₁-C₆, F, Cl, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR⁹, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où
R⁹ est H, Li, Na, K, 1/2Mg, 1/2Ca, des ions ammonium non substitués ou mono-, di- ou trisubstitués par alkyle en C₁-C₄, ou est un groupe alkyle en C₁-C₈,
R¹⁰ et R¹¹ représentent chacun indépendamment H, un groupe alkyle en C₁-C₅, phényle ou CH₂-phényle, le radical phényle étant non substitué ou mono-, di- ou trisubstitué par F, Cl, Br, I, alkyle en C₁-C₄, O-alkyle en C₁-C₄ ou CF₃ ;
ou
R¹⁰ et R¹¹ forment ensemble un groupe alkylène en C₄-C₅, dans lequel un groupe CH₂ peut être remplacé par O, S, NH, N-CH₃ ou N-benzyle ;
R³ est H, un groupe alkyle en C₁-C₆, F, Cl, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR⁹, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où R⁹, R¹⁰ et R¹¹ sont chacun tels que définis plus haut.

4. Procédé pour la préparation des composés de formule IV selon les revendications 1 à 3, dans laquelle :
R1 est H ;
R² est H ;
R³ est H, un groupe alkyle en C₁-C₆, F, Cl, Br, I, un groupe O-alkylène(C₀-C₈)-H, CF₃, OCF₃, SCF₃, SF₅, OCF₂-CHF₂, aryle en C₆-C₁₀, O-aryle en C₆-C₁₀, O-alkylène(C₁-C₄)-aryle(C₆-C₁₀), NO₂, COOR⁹, CONR¹⁰R¹¹, SH, ou NR¹⁰R¹¹, le radical aryle étant non substitué ou mono-, di- ou trisubstitué par F, Cl, Br, I, alkyle en C₁-C₄, O-alkyle(C₁-C₄) ou CF₃ ;
où
R⁹ est H, Li, Na, K, 1/2Mg, 1/2Ca, des ions ammonium non substitués ou mono-, di- ou trisubstitués par alkyle en C₁-C₄, ou est un groupe alkyle en C₁-C₈,
R¹⁰ et R¹¹ représentent chacun indépendamment
H, un groupe alkyle en C₁-C₅, phényle ou CH₂-phényle, le radical phényle étant non substitué ou mono-, di- ou trisubstitué par F, CI, Br, I, alkyle en C₁-C₄, O-alkyle en C₁-C₄ ou CF₃ ; ou
R¹⁰ et R¹¹ forment ensemble un groupe alkylène en C₄-C₅, dans lequel un groupe CH₂ peut être remplacé par O, S, NH, N-CH₃ ou N-benzyle.

5. Procédé pour la préparation des composés de formule IV selon les revendications 1 à 4, dans lesquels :
W = CH ;
o = 1 ;
R¹ = H;
R² = H, CH₃, OCH₃, Br ou Cl;
R³ = H, CH₃, OCH₃, Br ou Cl;
R⁴ = CH₃, CH₂CH₃ ou CH(CH₃)₂ ;
R⁵ = H, CH₃, CH₂CH₃ ou CH(CH₃)₂ ;
R⁶ = H, CH₃, CH₂CH₃ ou CH(CH₃)₂ ;
X³ = Cl, CH₃SO₃ ou p-CH₃-C₆H₄-SO₃ et
n2 = 0 ou 1.

6. Procédé pour la préparation des composés de formule IV selon la revendication 1, les composés de formule IV étant de préférence définis par la formule VIII 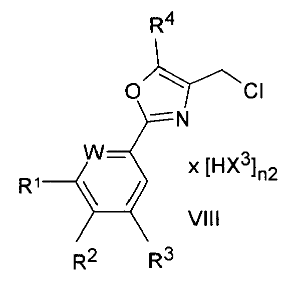 où
R¹ = H,
R2 = H ou CH₃,
R3 = H ou OCH₃, R⁴ = CH₃ ou CH(CH₃)₂,
W = CH,
X³ = Cl ou CH₃SO₃ et
n2 = 0 ou 1.

7. Procédé pour la préparation des composés de formules IV ou VIII selon les revendications 1 à 6, dans lequel la température de réaction pour la formation des N-oxydes (formule III) à partir de composés de formules I et II est comprise entre -20 °C et +150 °C.

8. Procédé pour la préparation des composés de formules IV ou VIII selon les revendications 1 à 7, dans lequel la réaction pour la formation des N-oxydes (formule III) est effectuée dans des solvants protiques, polaires, aprotiques, non polaires ou des mélanges des solvants mentionnés.

9. Procédé pour la préparation des composés de formules IV ou VIII selon les revendications 1 à 8, dans lequel la température de réaction pour la formation des halogénométhyloxazoles (formule IV) à partir de composés de formule III est dans la plage allant de -20 °C à +150 °C.

10. Procédé pour la préparation des composés de formule IV ou VIII selon les revendications 1 à 9, dans lequel la réaction pour la formation des halogénométhyloxazoles (formule IV) est effectuée dans des solvants aprotiques dipolaires, aprotiques polaires, aprotiques non polaires ou des mélanges des solvants mentionnés.

11. Procédé pour la préparation des composés de formules IV ou VIII selon les revendications 1 à 10, dans lequel le composé de formule R⁸X² est utilisé en un excès de 1 à 4x par rapport au N-oxyde (formule III).

12. Utilisation d'un ou plusieurs des composés de formule I, II, III ou IV dans un procédé pour la préparation des composés de formule IV ou VIII selon les revendications 1 à 11.
